## Europäisches Patentamt
## European Patent Office
## Office européen des brevets

(19)

(11) Publication number: **0 006 924**

**B1**

(12)

# EUROPEAN PATENT SPECIFICATION

(45) Date of publication of patent specification: **17.06.81**

(51) Int. Cl.³: **A 61 F 7/08**

(21) Application number: **78900146.8**

(22) Date of filing: **05.10.78**

(86) International application number:
**PCT/SE78/00052**

(87) International publication number:
**WO 79/00190 19.04.79 Gazette 79/8**

(54) Heating pad.

(30) Priority: **06.10.77 DK 4421/77**
**14.11.77 DK 4148/77**

(43) Date of publication of application:
**23.01.80 Bulletin 80/2**

(45) Publication of the grant of the European patent:
**17.06.81 Bulletin 81/24**

(84) Designated Contracting States:
**CH DE FR GB LU SE**

(56) References cited:
**DE - A - 1 429 375**
**DE - A - 2 307 938**
**SE - B - 395 139**
**US - A - 3 258 791**

(73) Proprietor: **PETERSEN, Kurt**
**Kongebakken 18**
**DK-5700 Svendborg (DK)**

(72) Inventor: **PETERSEN, Kurt**
**Kongebakken 18**
**DK-5700 Svendborg (DK)**

(74) Representative: **Paget, Hugh Charles Edward et al,**
**MEWBURN ELLIS & CO. 70/72 Chancery Lane**
**London WC2A 1AD (GB)**

Courier Press, Leamington Spa, England.

Heating pad

The present invention relates to a heating pad intended in particular for patients suffering from rheumatic and other ailments which make it necessary for some parts of the body to be kept warm.

Electric heating pads, in particular, have hitherto been used for that purpose. Such pads, however, cannot be used when the patients are out of doors or when they are moving around. One has to use, instead, an insulating material of one kind or another, e.g. camel-hair articles, but one cannot thereby keep a sufficiently high temperature of the skin in the affected regions.

Mattresses and sitting pads provided with inserts of foamed plastics for protection from cold are known, cf. for instance the German OS No. 2 033 981 and No. 2 307 938. The inventor has, in connection with the development of the present invention, examined whether this material could be used for heating pads to be carried on the body, but it has proved that said material does not give an adequate thermal effect.

It has now been found, according to the invention, that an adequate thermal effect can be achieved by use of a special insulating material which is characterized by a great number of air- or gas-filled bubbles or small bodies.

The heating pad according to the invention, which is based upon this recognition, consists of an envelope provided with an insert of insulating material, and the characteristic feature of the invention is that the insert consists of one or more flexible sheets of plastics material, each sheet being provided on one or both of its faces with a plurality of protruberances of a plastics material wherein each protruberance is a closed air- or gas-filled bubble or body and a plurality of such protruberances is present on at least one of the sheet faces forming an outer surface of the insert.

When the heating pad is being used, warm air will be stored in these small bubbles, and the heating pad will consequently be in a position to retain the body heat to such an extent that, under the pad, a skin temperature of e.g. up to 36.5°C can be reached.

The surface area of the air-filled pattern can advantageously amount to at least 20% of the surface of the sheets.

When use is made of a plurality of sheets, these are preferably welded together at their edges, so as to constitute an insert consisting of coherent sheets.

The envelope is made preferably of soft fabric, e.g. flannel, and is preferably detachable, e.g. provided with a "bur"-type fastener.

A particularly suitable heating pad consists, according to the invention, of two or more interconnected thin, flexible plastics sheets each of which on one face is provided with a plurality of evenly distributed, preferably circular, hollow air-filled protruberances of soft plastics material.

Such a material is manufactured, for instance, by the firm of NPE Emballage A/S, Haslev, Denmark.

U.S.A. Patent No. 3,577,305 discloses an insulating structure for incorporation in clothing etc. for use in cold weather. The structure described consists of one or more layers of sealed air cell material in which each of the sealed cells projects towards the interior of the structure and contacts a reflective sheet. This arrangement is the opposite to that in the present invention in which it is essential that the protruberances project outwardly from at least one of the outer surfaces of the sheets. U.S.A. 3,577,305 emphasises the importance of the smoothness of the outer surfaces of the structure described and is not concerned with the use of the structure as an insert for a heating pad.

In the drawing.

Fig. 1 shows schematically, on a reduced scale, an embodiment of the heating pad according to the invention seen from the face intended for contact with the body of the user, a corner of the envelope having been removed so as to make it possible to see the upper insert sheet, and

Fig. 2 shows schematically, on a reduced scale, the same embodiment in perspective, a corner being shown open so as to allow a view of the insert which is also open.

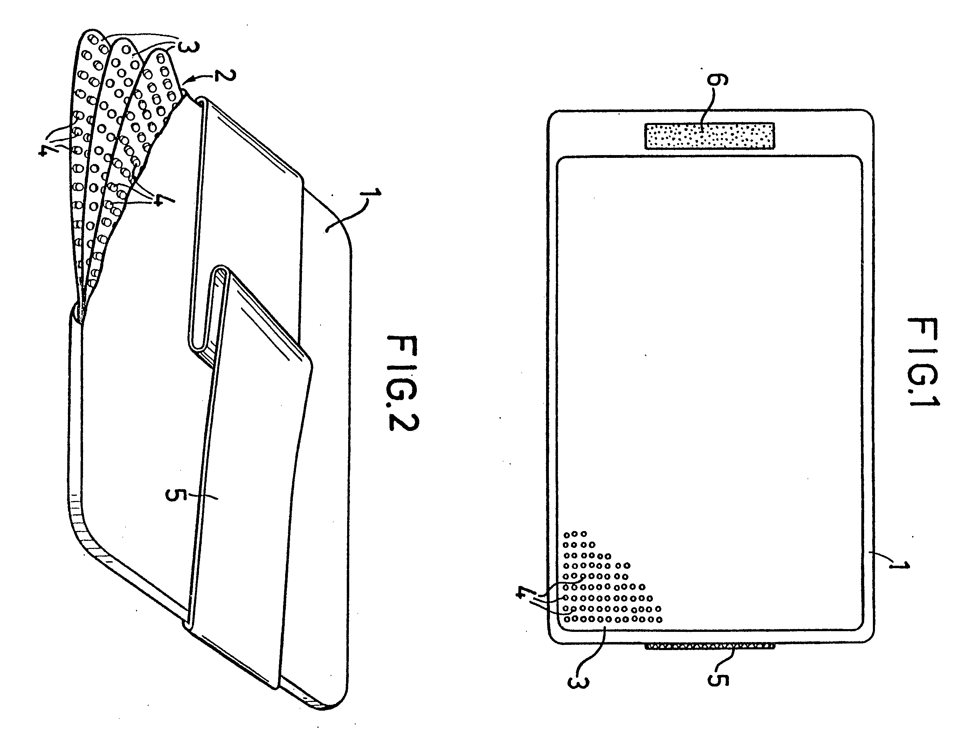

In the drawing, 1 is an envelope, e.g. of flannel. 2 (Fig. 2) is an inset consisting of thin, flexible, transparent plastics sheets 3 which are provided on one face with a great number of protuberances 4 evenly distributed, consisting of air-filled bubbles of thin soft plastics material. The sheets are welded together at their edges. The sheets are disposed so that one outer surface—the one facing the body of the user—shows protuberances, while the other outer surface is smooth. The heating pad is provided with a belt 5 of elastic material, whereby the pad can be fastened in the desired position.,

6 (Fig. 1) represents a "bur"-type fastener, e.g. the fastener sold under the trade name of Velcro.

In the embodiment shown, the surface area of the soft air-filled protuberances constitutes about 25% of the sheet area. Their height, in natural size, is about 2 mm.

In order to attain a suitably high skin temperature, it has proved sufficient for the insert to have a total thickness of just under 1 cm, and since the sheets are extremely light, the heating pad is very pleasant to wear and does not hamper the working ability.

It has also to be mentioned that a pattern as the one shown allows an adequate ventilation between the heating pad and the skin, which contributes to the comfort in use.

The heating pad according to the invention may, of course, have any shape and size, as it can be intended for use in different regions of the body, and it can also form part of garments, e.g. be inserted in the shoulder region, the back and/or the front part of a waistcoat.

## Claims

1. A heating pad consisting of an envelope (1) provided with an insert (2) of insulating material, characterized in that the insert (2) consists of one or more flexible sheets (3) of plastics material, each sheet having on one or both of its faces a plurality of protruberances of a plastics material wherein each protruberance is a closed air- or gas-filled bubble or body (4) and a plurality of such protruberances is present on at least one of the sheet faces forming an outer surface of the insert.

2. A heating pad according to claim 1 wherein the insert (2) consists of two or more interconnected thin, flexible sheets (3) each of which, on one face is provided with a plurality of evenly distributed, preferably circular, hollow air-filled protruberances (4) of soft plastics material.

## Revendications

1. Coussin thermique se composant d'une enveloppe (1) munie d'une garniture (2) en matière isolante, caractérisé en ce que la garniture (2) est constituée par une ou plusieurs plaques (3) flexibles en matière plastique, chaque plaque présentant, sur une face ou sur ses deux faces, un grand nombre de protubérances en matière plastique, chaque protubérance étant une bulle ou corps (4) fermé, rempli d'air ou de gaz, et un grand nombre de ces protubérances étant disposées sur une au moins des faces de plaque formant une surface externe de la garniture.

2. Coussin thermique selon la revendication 1, dans lequel la garniture (2) est constituée par deux ou plusieurs plaques (3) minces, flexibles, reliées mutuellement, chacune de ces plaques présentant sur une face un grand nombre de protubérances (4) en matière plastique douce, creuses, remplies d'air, de préférence circulaires, réparties uniformément.

## Patentansprüche

1. Thermokissen, bestehend aus einem Bezug (1) mit einer Einlage (2) aus isolierendem Material, dadurch gekennzeichnet, dass die Einlage aus einer oder mehreren biegsamen Schichten (3) aus Plast besteht, die je auf der einen Seite oder auf beiden Seiten eine Mehrzahl von Vorsprüngen aus Plast besitzen, wobei jeder Vorsprung eine geschlossene luft- oder gasgefüllte Blase oder ein geschlossener luft- oder gasgefüllter Kleinkörper (4) ist, und eine Mehrzahl von solchen Vorsprüngen an mindestens einer der Seiten der Schicht, die eine Aussenseite der Einlage ausmacht, vorhanden ist.

2. Thermokissen nach Anspruch 1, dadurch gekennzeichnet, dass die Einlage (2) aus zwei oder mehreren untereinander verbundenen, dünnen, biegsamen Schichten (3) besteht, die je auf deren einen Seite mit einer Mehrzahl gleichmässig verteilter, vorzugsweise zirkularer, hohler luftgefüllter Vorsprünge (4) aus weichem Plast vorgesehen ist.

# FIG.1

# FIG.2